# EUROPEAN PATENT SPECIFICATION

(11) **EP 1 703 859 B1**
(45) Date of publication and mention of the grant of the patent: **20.04.2011**
(21) Application number: 04811653.7
(22) Date of filing: 19.11.2004
(51) Int. Cl.: A61F 2/82

(54) **STENT TO BE DEPLOYED ON A BEND**
STENT ZUR ABLAGE AUF EINER BIEGUNG
STENT A DEPLOYER SUR UNE COURBE

(30) Priority: 30.12.2003 US 749170
(43) Date of publication of application: 27.09.2006
(73) Proprietor: Boston Scientific Limited, Barbados, West Indies (BB)
(72) Inventor: GREGORICH, Daniel, St. Louis Park, MN 55416 (US)
(74) Representative: Hauck Patent- und Rechtsanwälte
(86) International application number: PCT/US2004/038964
(87) International publication number: WO 2005/065580

(56) References cited:
- EP-A- 1 304 091
- WO-A-02/060344
- US-A1- 2002 007 212
- US-B1- 6 503 270

## Description

### Background of the Invention

Stents are placed or implanted within a variety of bodily vessels including in coronary arteries, renal arteries, peripheral arteries including illiac arteries, arteries of the neck and cerebral arteries, veins, biliary ducts, urethras, fallopian tubes, bronchial tubes, the trachea, the esophagus and the prostrate.

Stents are available in a wide range of designs. One popular stent design includes a plurality of serpentine rings having alternating turns. The rings are made of interconnected struts. Adjacent rings are interconnected via connecting elements.

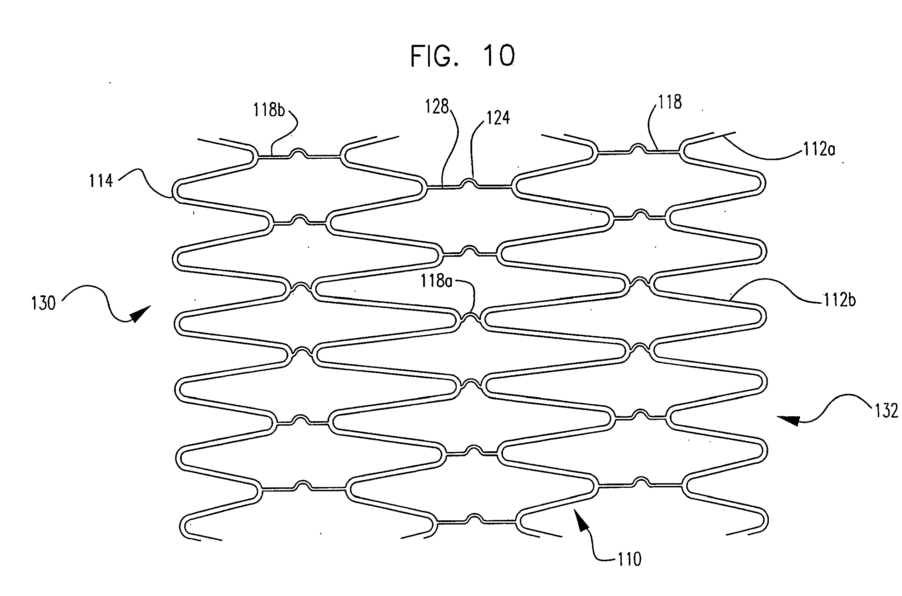

US 2002/0007212 A1 discloses a longitudinally flexible expandable stent. The stent comprises a plurality of undulating band-like elements wherein each band-like element has alternating peaks and troughs. The embodiment shown in figure 10 discloses a stent wherein each band-like element comprises peaks and troughs of the same amplitude which are grouped together within the band-like element.

Generally, when stents are deployed in areas of high curvature they are bent so that the struts on the outside of the curve are farther apart than those on the inside of the curve. This arrangement typically provides poor scaffolding on the outside of the bend and/or possibly result in overlapping struts on the inside of the bend.

The technical problem is to provide a stent having adequate scaffolding in areas of high curvature.

The problem is solved according to claim 1.

### Summary of the Invention

According to the invention the stent comprises a first segment having a plurality of closed serpentine circumferential bands. Adjacent closed serpentine circumferential bands are connected to one another. Each closed serpentine circumferential band has a plurality of struts, each strut having a length, and the struts which are circumferentially adjacent to one another are connected one to the other by a turn. The struts generally increase in length from a minimum strut length to a maximum strut length, and then generally decrease in length from the maximum strut length to the minimum strut length as the circumferential band is traversed in its entirety in a clockwise direction. Desirably, the struts of maximum length in the closed serpentine bands may be generally longitudinally aligned with one another.

Embodiments which characterize the invention are pointed out with particularity in the claims annexed hereto and forming a part hereof.

However, for a better understanding of the invention, its advantages and objectives obtained by its use, reference should be made to the drawings which form a further part hereof and the accompanying descriptive matter, in which there are illustrated and described various embodiments of the invention.

### Brief Description of the Figures

Figure 1 shows a flat pattern design of an embodiment of an inventive stent.
Figure 2 shows another flat pattern design of an embodiment of an inventive stent.
Figure 3 shows another flat pattern design of an embodiment of an inventive stent.
Figure 4 shows another flat pattern design of an embodiment of an inventive stent.
Figure 5 shows another flat pattern design of an embodiment of an inventive stent.
Figure 6 shows another flat pattern design of an embodiment of an inventive stent.
Figure 7 shows another flat pattern design of an embodiment of an inventive stent.
Figure 8 shows another flat pattern design of an embodiment of an inventive stent.
Figure 9 shows another flat pattern design of an embodiment of an inventive stent.
Figure 10 shows another flat pattern design of an embodiment of an inventive stent.
Figure 11 shows another flat pattern design of an embodiment of an inventive stent.
Figure 12 shows another flat pattern design of an embodiment of an inventive stent.
Figure 13 shows an embodiment of an inventive stent deployed in a curved vessel.

### Detailed Description of the Invention

This invention may be embodied in many different forms. This description is an exemplification of the principles of the invention and is not intended to limit the invention to the particular embodiments illustrated.

For the purposes of this disclosure, unless otherwise indicated, identical reference numerals used in different figures refer to the same component.

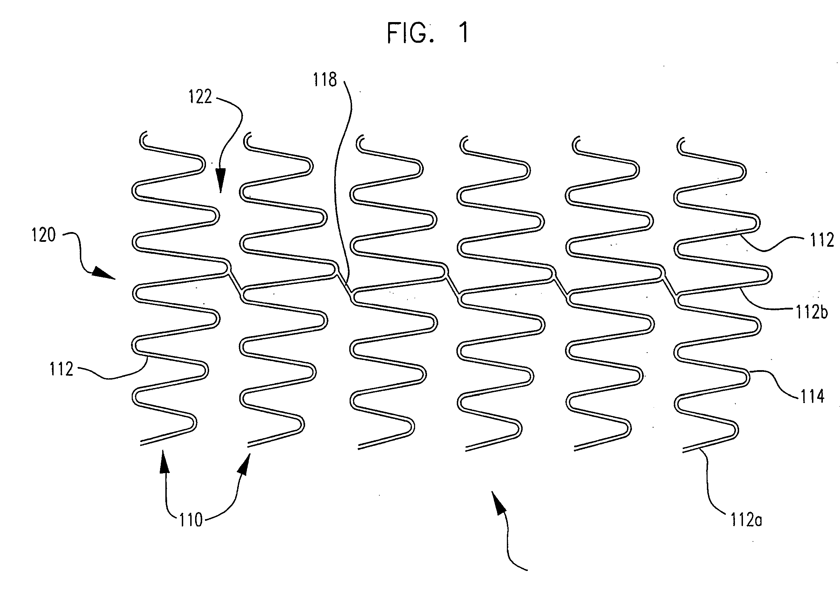

In one embodiment, the invention is directed to a stent, such as that shown at 100 in Fig. 1, comprising a plurality of interconnected closed serpentine circumferential bands 110. Adjacent closed serpentine circumferential bands 110 may be connected to one another by at least one connecting element 118. Each closed serpentine circumferential band 110 comprises a plurality of struts 112. Struts 112 which are circumferentially adjacent to one another are connected to one another by a turn 114. The length of the struts 112 within a band 110 generally increases in length from a minimum strut length to a maximum strut length and then generally decreases in length from the maximum length to the minimum length as the circumferential band is traversed in its entirety in a clockwise direction. A strut of maximum length is shown at 112b while a strut of minimum length is shown at 112a. The term 'generally increasing' allows for the possibility that some adjacent struts 112 are of the same length or within manufacturing tolerances of one another. Similarly, the term 'generally decreasing' allows for the possibility that some adjacent struts 112 are of the same length or within manufacturing tolerances of one another. Desirably, the struts 112 continually increase in length over a portion of the circumferential band 110 and then continually decrease in length over the remaining portion of the circumferential band 110. The term 'continually increase' requires that each strut 112 in the direction of increasing length be longer than the previous strut 112. The term 'continually decrease' requires that each strut 112 in the direction of decreasing length be shorter than the previous strut 112. Adjacent bands 110 are connected one to the other via one or more connectors 116.

Each band of the stent will have a distribution of struts ranging from a smallest strut to a largest strut. The distribution of struts may be the same in each band or may differ in one or more of the bands. Thus, the maximum strut length of the struts of a first circumferential band may or may not be the same as the maximum strut length of the struts of a second circumferential band. Similarly, the minimum strut length of the struts of a first circumferential band may or may not be the same as the minimum strut length of the struts of the second circumferential band. In some embodiments, the number of struts per band may differ between some of the bands. Also, the range of strut width within a band may differ among some of the bands.

Typically, as shown in Fig. 1, the struts of maximum length 112b in the closed serpentine circumferential bands 110 are generally longitudinally aligned with one another. For the purposes of this disclosure, two struts 112 are in general longitudinal alignment with each other if a line which is parallel to the longitudinal axis of the stent can be drawn between the two struts 112. The line may be straight in the case of a stent without curvature or may be curved in the case of a stent with curvature.

As shown in Fig. 1, each closed serpentine circumferential band 110 has a first end 120 and a second end 122, and the turns 114 at only one of the first and second ends are in general circumferential alignment. The turns 114 at the other end are generally not in circumferential alignment. For the purposes of the disclosure, turns 114 are in circumferential alignment if they extend to the same longitudinal extent along the length of the stent 100. All of the bands 110 may be similarly oriented as shown in Fig. 1 so that the unaligned end of one band 110 faces the aligned end of a directly adjacent band 110.

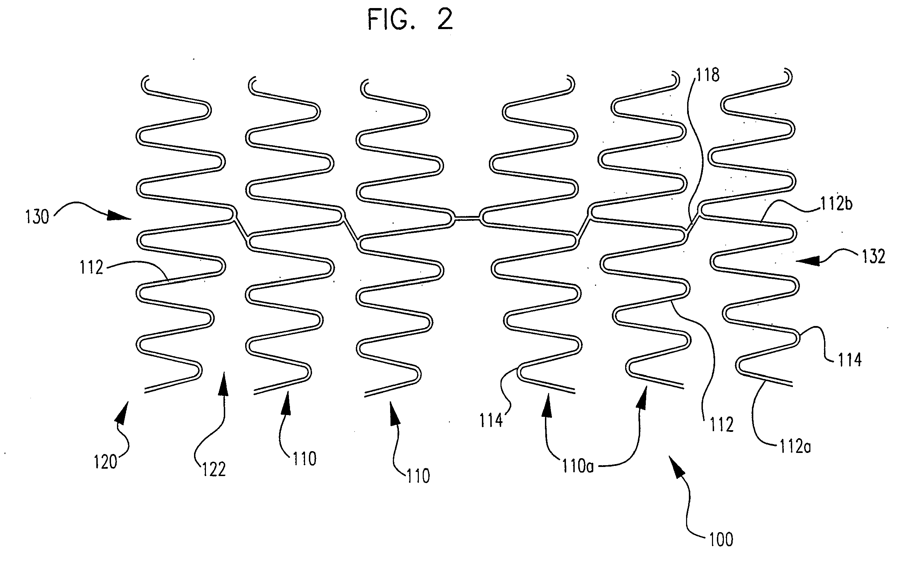

It is also within the scope of the invention, as shown in Fig. 2, for some of the bands 110 to be oriented in one direction and for other bands 110a to be oriented in the opposite direction. For example, in the stent 100 of Fig. 2, the bands 110 located toward the proximal end 130 of the stent 100 have unaligned second ends 122, while the bands 110a located toward the distal end 132 of the stent have unaligned first ends 120. As shown, bands 110 and bands 110a are oppositely oriented. Thus, in the middle of the stent 100, there may be a first serpentine band 110 adjacent to a second serpentine band 110a, wherein the non-aligned turns 114 of the first serpentine band 110 face the non-aligned turns 114 of the second serpentine band 110a.

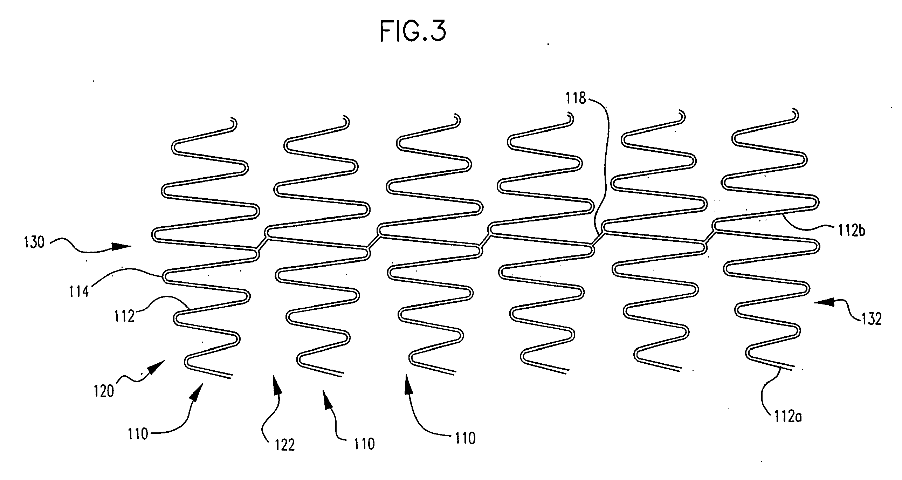

It is further within the scope of the invention for the closed serpentine bands 110 to have non-aligned turns 114 at both the first end 120 and the second end 122, as shown by way of example in Fig. 3.

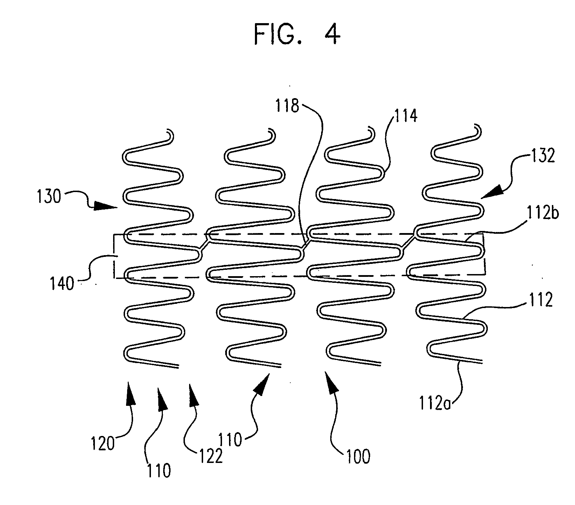

In another embodiment as shown at 100 in Fig. 4, the invention is directed to a stent comprising a plurality of interconnected serpentine circumferential bands 110. Adjacent closed serpentine circumferential bands 110 may be connected to one another by at least one connecting element 118.

Each serpentine circumferential band 110 may have a first end 120 and a second end 122, and may comprise a plurality of struts 112. Struts 112 which are circumferentially adjacent to one another are connected to one another by a turn 114. The length of the struts 112 within a band 110 generally increases in length from a minimum strut length 112a to a maximum strut length 112b and then generally decreases in length from the maximum length 112b to the minimum length 112a as the circumferential band 110 is traversed in its entirety in a clockwise direction.

Each circumferential band 110 within a stent 100 may have struts 112 that vary in length as compared to other circumferential bands 110 within the stent 100. Thus, the maximum strut length 112b of a first circumferential band 110 may or may not be the same as the maximum strut length 112b of a second circumferential band 110. Similarly, the minimum strut length 112a of a first circumferential band 110 may or may not be the same as the minimum strut length 112a of a second circumferential band 110. In some embodiments, the maximum strut length 112b of a first circumferential band 110 may be the same as or even shorter than the minimum strut length 112a of a second circumferential band 110.

As shown in Figure 4, all of the turns 114 at the proximal end 130 of a stent 100 may be in general circumferential alignment with one another. All of the turns 114 at the distal end 132 of a stent 100 may be in general circumferential alignment with one another. Interior turns 114, which are not located at either the proximal end 130 or the distal end 132 of the stent 100, may be out of circumferential alignment with other interior turns 114 that are located on the same side 120, 122 of the same serpentine circumferential band 110.

As shown in Figure 4, an inventive stent 100 may include a strip or backbone section 140. Desirably, a strip 140 comprises a zone extending across at least a portion of the length of the stent 100 and across at least a portion of the circumference of the stent 100. In some embodiments, all struts 112 located within a strip 140 may comprise maximum length struts 112b. In some embodiments, a strip 140 may contain all of the maximum length struts 112b included in a stent 100.

In some embodiments, such as shown in Figure 4, a strip may include all of the connecting elements 118 of the stent 100.

When an inventive stent 100 is deployed on a curve, desirably the stent 100 will be positioned having a strip 140 located at the outside of the curve.

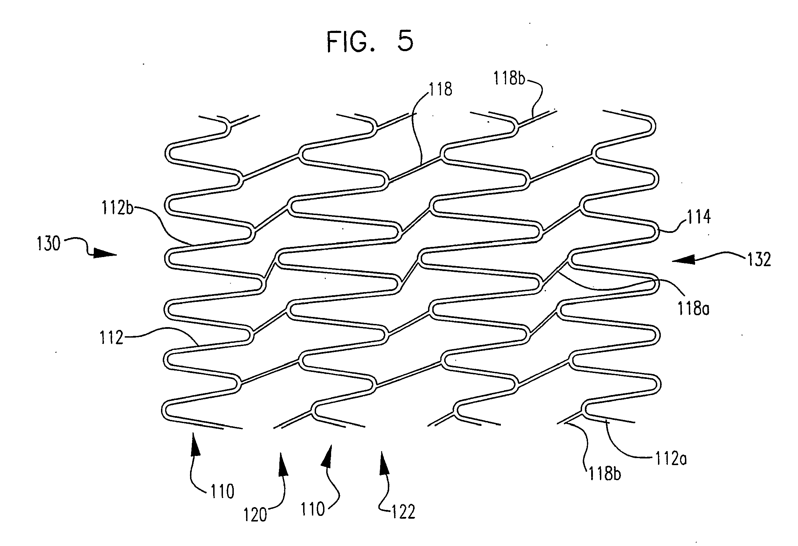

In another embodiment as shown at 100 in Fig. 5, the invention is directed to a stent comprising a plurality of interconnected serpentine circumferential bands 110. Adjacent closed serpentine circumferential bands 110 may be connected to one another by a plurality of connecting elements 118.

Each serpentine circumferential band 110 may have a first end 120 and a second end 122, and may comprise a plurality of struts 112. Struts 112 which are circumferentially adjacent to one another are connected to one another by a turn 114. The length of the struts 112 within a band 110 generally increases in length from a minimum strut length 112a to a maximum strut length 112b and then generally decreases in length from the maximum length 112b to the minimum length 112a as the circumferential band 110 is traversed in its entirety in a clockwise direction.

Each circumferential band 110 within a stent 100 may have struts 112 that vary in length as compared to other circumferential bands 110 within the stent 100. Thus, the maximum strut length 112b of a first circumferential band 110 may or may not be the same as the maximum strut length 112b of a second circumferential band 110. Similarly, the minimum strut length 112a of a first circumferential band 110 may or may not be the same as the minimum strut length 112a of a second circumferential band 110. In some embodiments, the maximum strut length 112b of a first circumferential band 110 may be the same as or even shorter than the minimum strut length 112a of a second circumferential band 110.

All of the turns 114 at the proximal end 130 of a stent 100 may be in general circumferential alignment with one another. All of the turns 114 at the distal end 132 of a stent 100 may be in general circumferential alignment with one another. Interior turns 114, which are not located at either the proximal end 130 or the distal end 132 of the stent 100, may be out of circumferential alignment with other interior turns 114 that are located on the same side 120, 122 of the same serpentine circumferential band 110.

Each connecting element 118 may be connected at one end to a turn 114 of a first serpentine circumferential band 110 and may be connected at the other end to a turn 114 of an adjacent serpentine circumferential band 110. As shown in Figure 5, the length of circumferentially adjacent connecting elements 118 may vary. In some embodiments, the length of the connector struts 118 between two adjacent serpentine circumferential bands 110 may generally increase in length from a minimum connecting element length 118a to a maximum connecting element length 118b, and then generally decrease in length from the maximum connecting element length 118b to the minimum connecting element length 118a about the circumference of the stent 100. In some embodiments, the length of the connecting elements 118 may be inversely proportional to the length of struts 112 located adjacent to the connecting elements 118.

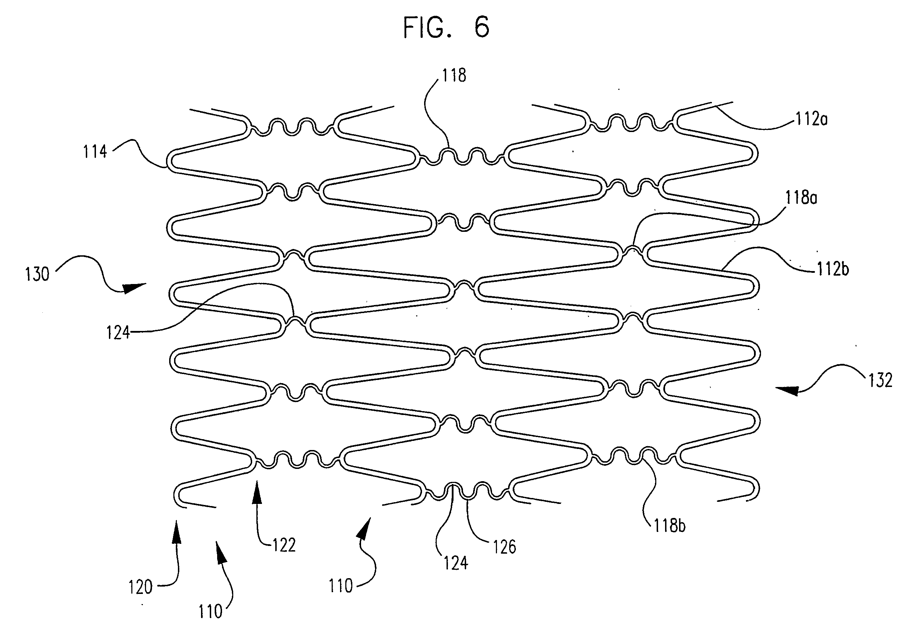

In another embodiment as shown at 100 in Fig. 6, the invention is directed to a stent comprising a plurality of interconnected serpentine circumferential bands 110. Adjacent closed serpentine circumferential bands 110 may be connected to one another by a plurality of connecting elements 118.

Each serpentine circumferential band 110 may have a first end 120 and a second end 122, and may comprise a plurality of struts 112. Struts 112 which are circumferentially adjacent to one another are connected to one another by a turn 114. The length of the struts 112 within a band 110 generally increases in length from a minimum strut length 112a to a maximum strut length 112b and then generally decreases in length from the maximum length 112b to the minimum length 112a as the circumferential band 110 is traversed in its entirety in a clockwise direction.

Each circumferential band 110 within a stent 100 may have struts 112 that vary in length as compared to other circumferential bands 110 within the stent 100. Thus, the maximum strut length 112b of a first circumferential band 110 may or may not be the same as the maximum strut length 112b of a second circumferential band 110. Similarly, the minimum strut length 112a of a first circumferential band 110 may or may not be the same as the minimum strut length 112a of a second circumferential band 110. In some embodiments, the maximum strut length 112b of a first circumferential band 110 may be the same as or even shorter than the minimum strut length 112a of a second circumferential band 110.

All of the turns 114 at the proximal end 130 of a stent 100 may be in general circumferential alignment with one another. All of the turns 114 at the distal end 132 of a stent 100 maybe in general circumferential alignment with one another. Interior turns 114, which are not located at either the proximal end 130 or the distal end 132 of the stent 100, may be out of circumferential alignment with other interior turns 114 that are located on the same side 120, 122 of the same serpentine circumferential band 110.

Each connecting element 118 may be connected at one end to a turn 114 of a first serpentine circumferential band 110 and may be connected at the other end to a turn 114 of an adj acent serpentine circumferential band 110. Each connecting element 118 may include curvature, and thus may include a peak 124. The length of a connecting element 118 may vary from the length of a circumferentially adjacent connecting element 118. Longer connecting elements 118 may further include a trough 126. Generally, a peak 124 may be connected via an inflection point to a trough 126. Still longer connecting elements 118 may include a plurality of peaks 124, and may also include at least one trough 126 or a plurality of troughs 126.

Curvature in a connecting element 118, such as peaks 124 and troughs 126, allow for changes in the span of the connecting element 118. A connecting element 118 that includes peaks 124 or troughs 126 may lengthen or foreshorten, for example during expansion of the stent 100. Thus, the distance between turns 114 to which the connecting element 118 is attached may be adjusted without sacrificing scaffolding support, and an inventive stent 100 may be adaptable for deployment within bodily lumens having varying degrees of curvature.

In some embodiments, the length of the connector struts 118 between two adjacent serpentine circumferential bands 110 may generally increase in length from a minimum connecting element length 118a to a maximum connecting element length 118b, and then generally decrease in length from the maximum connecting element length 118b to the minimum connecting element length 118a about the circumference of the stent 100. In some embodiments, the length of the connecting elements 118 may be inversely proportional to the length of struts 112 located adjacent to the connecting elements 118.

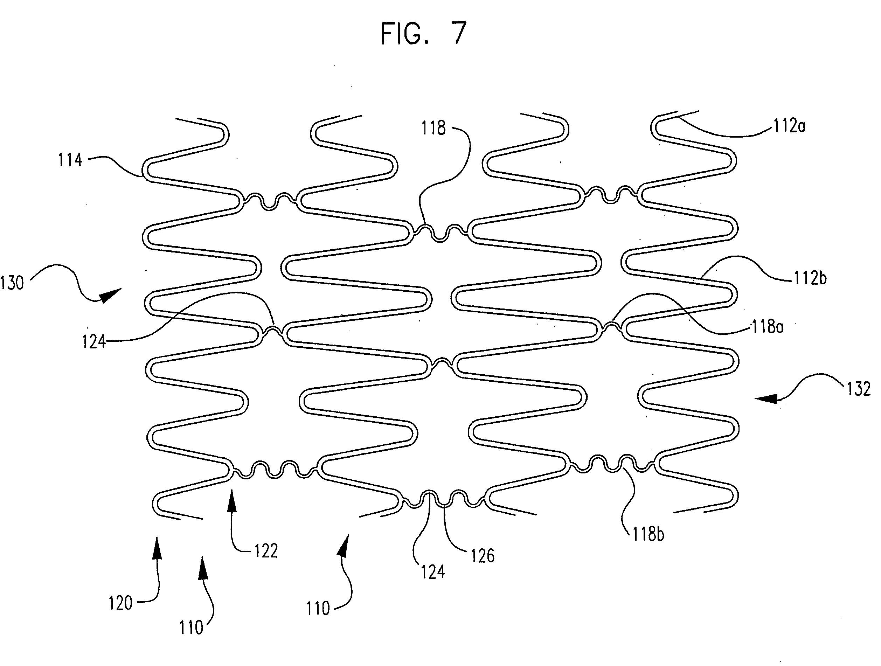

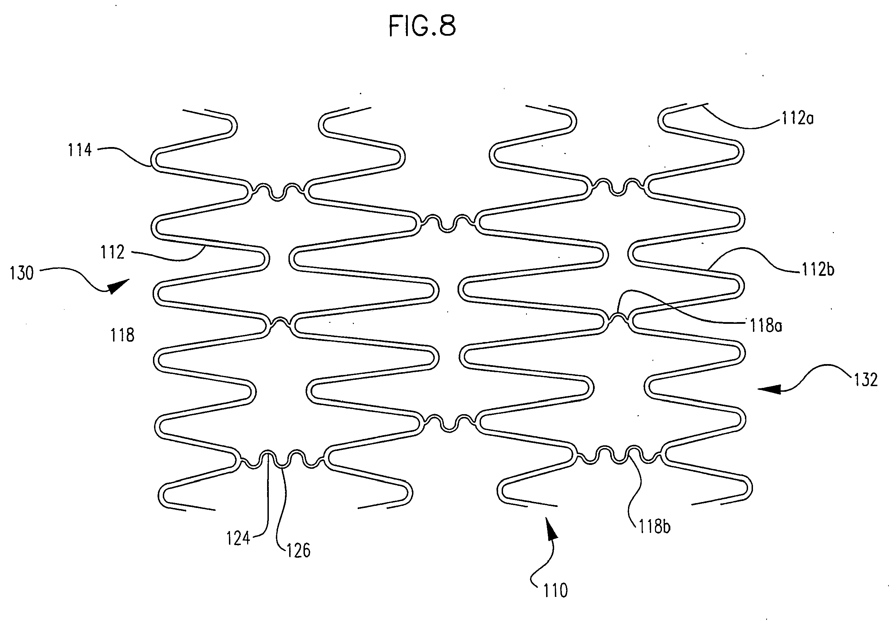

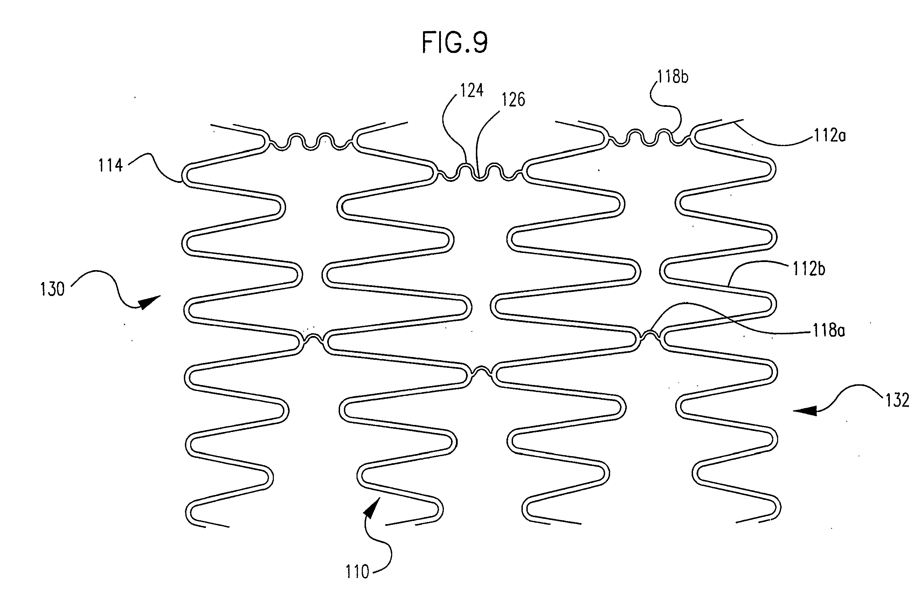

As shown in Figures 7 - 12, the number of connecting elements 118 and the shape of the connecting elements 118 may be varied without departing from the invention. Any number of connecting elements 118 may be used between adjacent serpentine circumferential bands 110. Connecting elements 118 may include peaks 124, troughs 126 or combinations of peaks 124 and troughs 126. Connecting elements 118 may span between turns 114 that are longitudinally aligned with one another, or may span diagonally between turns 114 that are not longitudinally aligned. Connecting elements 118 may further span between struts 112 of adj acent serpentine circumferential bands 110. Connecting elements 118 may have any suitable shape, cross-section or thickness.
Figures 7 - 9 show various embodiments of invention stents 100 having different connecting element 118 configurations, wherein the connecting elements 118 may include peaks 124, troughs 126 or combinations of peaks 124 and troughs 126.
Figure 10 shows an embodiment of an inventive stent 100. Connecting elements 118 may include a peak 124. The arc length and curvature of a peak 124 may be substantially uniform between all connecting elements 118 of the stent 100. Connecting elements 118 may also include one or more straight portions 128. The length of a straight portion may be dependent upon the span of the individual connecting element 118.

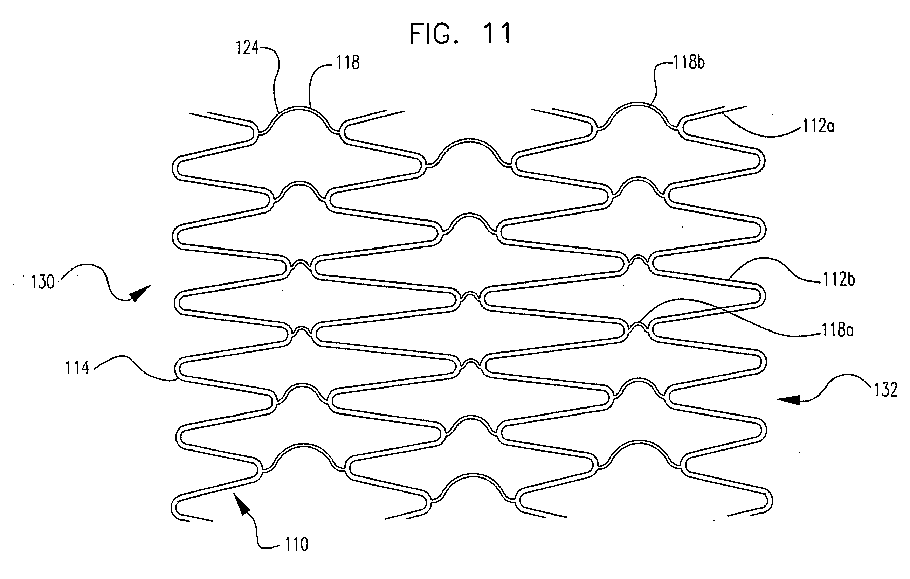

Figure 11 shows another embodiment of an inventive stent 100. Connecting elements 118 may be curved along their length. The curvature of all connecting elements 118 of the stent 100 may be substantially uniform. The length and span of connecting elements 118 may vary.

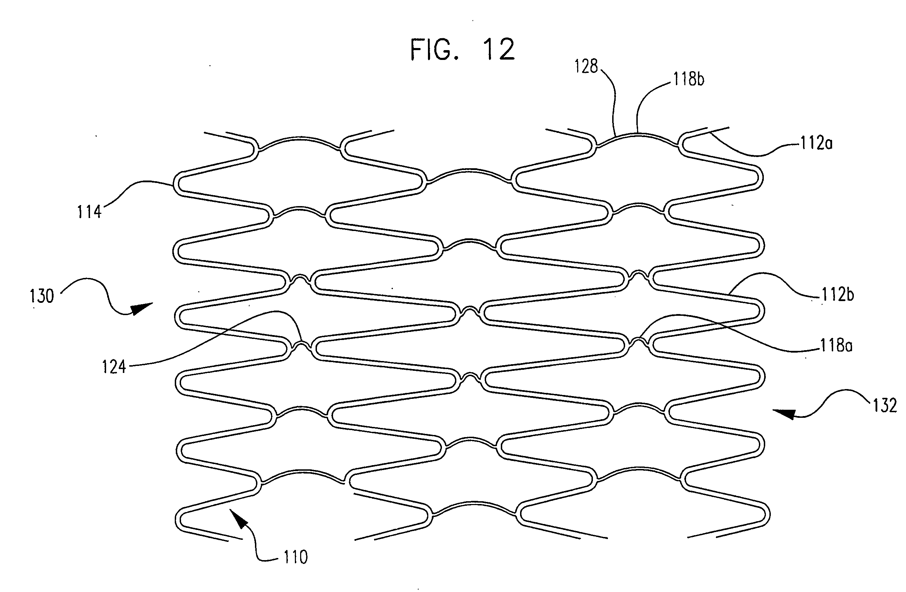

Figure 12 shows another embodiment of an inventive stent 100. Connecting elements 118 may be curved along portions of their length. Connecting elements 118 may include portions of semicircular or parabolic curvature. Connecting elements 118 may further include a straight portion 128. The length and span of connecting elements 118 may vary.

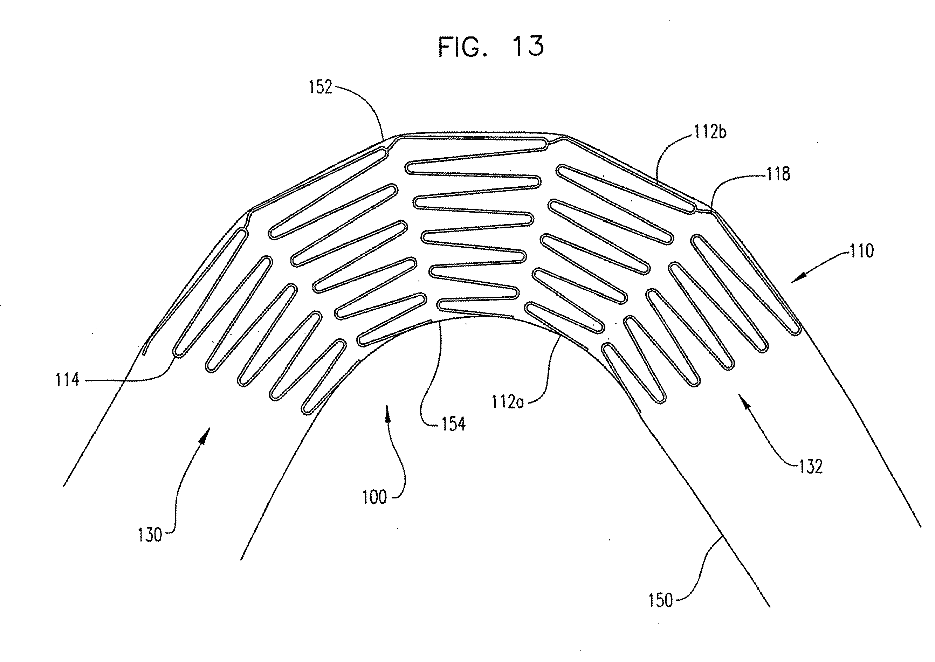

Figure 13 shows an embodiment of an inventive stent 100 deployed in a curved vessel 150. The curved vessel 150 may have an outside portion 152 and an inside portion 154. The stent 100 may be positioned such that the maximum length struts 112b support the outside portion 152 of the vessel 150.

In some embodiments, a stent 100 may be positioned such that a strip 140 is located against the outside portion 152 of a curved vessel 150.

In some embodiments, a stent 100 may be positioned such that the minimum length struts 112a support the inside portion 154 of the vessel 150.

The invention is also directed to an unexpanded stent 100 comprising a plurality of interconnected struts 112 disposed in a tubular structure where at least a portion of the tubular structure includes struts 112 which generally increase in length to a maximum length 112b and then generally decrease in length to a minimum length 112a as the stent 100 is traversed all the way about a longitudinal axis of the stent 100 in a circumferential direction. Examples of such stents 100 are shown in Figures 1 - 3. As shown in the Figures, the serpentine bands 110 are in general alignment with one another such that the struts 112 of maximum length in each band 110 are generally longitudinally aligned with one another.

The invention is also directed to a stent 100 comprising a plurality of interconnected struts 112 defining a wall surface. The wall surface may include a strip 140 extending from the proximal end 130 of the stent 100 to the distal end 132 of the stent 100 as shown in Figure 4. The strip 140 may extend over a portion of the circumference of the stent 100. The strip 140 is characterized as having a plurality of rows of interconnected struts 112 which are of greater length than the remaining struts 112 of the stent 100.

The invention is further directed to a stent 100 comprising a plurality of interconnected struts 112 defining a wall surface. The wall surface includes a strip 140 extending from proximal end 130 of the stent 100 to the distal end 132 of the stent 100. The strip 140 may extend over a portion of the circumference of the stent 100. The strip 140 may be characterized as having a plurality of rows of interconnected struts 112 which are of greater flexibility than the remaining struts 112 of the stent 100. In one embodiment, the greater flexibility is achieved via struts 112 which are longer than the remaining struts 112 of the stent 100. In another embodiment, the greater flexibility is achieved by having struts 112 which are thinner than the remaining struts 112 of the stent 100.

It is further within the scope of the invention to modify any of the stents 100 disclosed herein by providing the longer length struts 112 with wider widths or narrower widths as well, as compared with the shorter length struts 112. Thus, the width of the struts 112 may increase and then decrease along with the length of the struts 112.

It is further within the scope of the invention to modify any of the stents 100 disclosed herein by providing any of the embodiments of a serpentine band 110 along with any alternative embodiments of a serpentine band 110, all within the same inventive stent 100. For example, a stent 100 may include a first serpentine band 110 having aligned turns 114 at the first end 120 and unaligned turns 114 at the second end 122, and another serpentine band 110 having unaligned turns 114 at both the first end 120 and the second end 122.

Any of the inventive features described herein with respect to any of the disclosed embodiments may be selected and combined to form further embodiments of the invention.

In some embodiments, a stent 100 may include struts 112 that are parallel to the stent longitudinal axis when the stent is unexpanded.

Any of the inventive stents 100 described herein may include a strip 140 as described above.

Any of the inventive stents 100 described herein may be provided with portions of lesser or greater flexibility than other portions of the stent 100. For example, one or both ends of the stent 100 may be more flexible than the middle of the stent 100 or less flexible. Further, portions of a serpentine circumferential band 110 may be more or less flexible than other portions of the serpentine circumferential band 110. Changes in flexibility may be provided by adjustment of the length of struts 112 or the length and shape of connecting elements 118. Changes in flexibility may further be provided by adjusting the width, thickness and/or cross-sectional area of portions of serpentine circumferential bands 110 and/or connecting elements 118, by making them of weaker materials, or by any other suitable method.

All portions of any of the inventive stents 100 described herein may be provided with any cross-sectional shape, including square, rectangular, circular, ovular, triangular and/or trapezoidal cross sections.

Differences in flexibility may also be achieved by using any of the inventive stents disclosed herein as part of a stent containing other strut patterns as well. Thus, for example, it may be desirable to employ the stents disclosed herein in conjunction with more flexible stent segments of different geometry or less flexible stent segments of different geometry.

More generally, it may be desirable to employ the stents disclosed herein in conjunction with stent segments of different geometry in order to achieve other goals as well. Thus, the inventive stents disclosed herein may be used as a center portion of a stent containing segments of other geometries where only the center portion of the stent will be deployed in an area with a bend. Any known stent design may be used. Examples of particularly suitable stent designs are disclosed in US 20020055770, US 20020095208 and US 20020116049. It is also within the scope of the invention for the inventive stents disclosed herein to be used as an end segment of a stent.

The invention is also directed to stents 100 such as those disclosed herein arranged for sidebranch access. Such a stent 100 may be provided by omitting one more struts 112 and/or one or more turns 114 in one or more desired regions of the stent 100. Sidebranch access may also be provided by omitting a first serpentine band 110 and providing connecting elements 118 between some, but not all, of the turns 114 of the resulting adj acent serpentine bands 110. Sidebranch access may further be achieved in any of the inventive stents 100 disclosed herein by alternating the location of connecting elements 118 between adjacent serpentine bands 110. For example, where it is desirable to provide for sidebranch access, fewer connecting elements 118 between adjacent bands 110 may be provided. Any suitable combination of strut 112, turn 114 and/or connecting element 118, omissions thereof or modifications thereof may be used to provide sidebranch access. In some embodiments, omission of struts or connectors or modification of the stent 100 may be made within a strip 140.

The inventive stents 100 disclosed herein may also be used in bifurcated stents. The trunk and/or any of the branches may be provided with stents 100 having the novel designs disclosed herein. Any other stent of suitable design including those disclosed in US 20020055770, US 20020095208 and US 20020116049 may also be used in conjunction with the inventive stents disclosed herein to make a bifurcated stent.

Any of the inventive stents 100 disclosed herein may be provided with a uniform diameter or may taper in portions or along the entire length of the stent 100. Also, the width and/or thickness of the various portions of the inventive stents 100 may increase or decrease along a given portion of the stent 100. For example, the width and/or thickness of the serpentine bands 110 and/or connecting elements 118 may increase or decrease along portions of the stent 100 or along the entire length of the stent 100.

The inventive stents 100 may be manufactured using known stent manufacturing techniques. Suitable methods for manufacturing the inventive stents 100 include laser cutting, hybrid water-jet/laser cutting, chemical etching or stamping of a tube. The inventive stents 100 may also be manufactured by laser cutting, hybrid waterjet/laser cutting, chemically etching, or stamping a flat sheet, rolling the sheet and welding the sheet, by electrode discharge machining, or by molding the stent 100 with the desired design.

Any suitable stent material may be used in the manufacture of the inventive stents 100. Examples of such materials include polymeric materials, metals, ceramics and composites. Suitable polymeric materials include thermotropic liquid crystal polymers (LCP's). Where the stent 100 is made of metal, the metal may be stainless steel, cobalt chrome alloys such as elgiloy, tantalum or other plastically deformable metals. Other suitable metals include shape-memory metals such as nickel titanium alloys generically known as "Nitinol," platinum/tungsten alloys and titanium alloys. The invention also contemplates the use of more than one material in the inventive stents 100. For example, some serpentine bands 110 may be made of different materials than other serpentine bands 110 within the same stent 100. Optionally, the connecting elements 118 may be made of a different material than the first and/or second serpentine bands 110.

It is also within the scope of the invention for longer struts 112 to be made from a different material than the shorter struts 112, or for the longer struts 112 to be made the same material as the shorter struts 112, the material having been differently treated.

The inventive stents 100 desirably are provided in self-expanding form. To that end, they may be constructed from shape memory materials including Nitinol. The self-expanding embodiments of the invention allow for a controlled expansion of the stent 100 as explained below. Typically, self-expanding stents are restrained on a catheter in an unexpanded configuration via a sheath. As the sheath is withdrawn, the newly freed portions of the stent will self-expand. Because the individual turns 114 of a serpentine band 110 may be unaligned, and thus extend to different locations along the longitudinal axis of the stent while the stent is unexpanded and sheathed, each serpentine circumferential band 110 will expand in several waves - the first wave of turns 114, which may correspond to the turns 114 connected to the shortest length struts 112a, depending upon serpentine band 110 orientation, will expand first, followed by a wave of turns 114 which are connected to longer struts 112 expanding, and so forth until all of the turns 114 have opened.

The inventive stents 100 may also be provided in balloon expandable form, or as a hybrid, having self-expanding characteristics and balloon expandable characteristics.

The invention is also directed to the combination of an inventive stent disclosed herein and a catheter. The catheter may include a balloon for use with a balloon expandable stent and/or may include a restraining device to restrain the stent in the case of a self-expanding stent.

The inventive stents 100 may include suitable radiopaque coatings. For example, the stents may be coated with gold or other noble metals or sputtered with tantalum or other metals. The stents may also be made directly from a radiopaque material to obviate the need for a radiopaque coating or may be made of a material having a radiopaque inner core. Other radiopaque metals which may be used include platinum, platinum tungsten, palladium, platinum iridium, rhodium, tantalum, or alloys or composites of these metals.

The inventive stents 100 may also be provided with various biocompatible coatings to enhance various properties of the stent. For example, the inventive stents 100 may be provided with lubricious coatings. The inventive stents 100 may also be provided with drug-containing coatings which release drugs over time.

The inventive stents 100 may also be provided with a sugar or more generally a carbohydrate and/or a gelatin to maintain the stent on a balloon during delivery of the stent to a desired bodily location. Other suitable compounds for treating the stent include biodegradable polymers and polymers which are dissolvable in bodily fluids. Portions of the interior and/or exterior of the stent 100 may be coated or impregnated with the compound. Mechanical retention devices may also be used to maintain the stent on the balloon during delivery.

The inventive stents 100 may also be used as the framework for a graft. Suitable coverings include nylon, collagen, PTFE and expanded PTFE, polyethylene terephthalate and KEVLAR, or any of the materials disclosed in US 5,824,046 and US 5,755,770. More generally, any known graft material may be used including synthetic polymers such as polyethylene, polypropylene, polyurethane, polyglycolic acid, polyesters, polyamides, their mixtures, blends, copolymers, mixtures, blends and copolymers.

The inventive stents 100 may find use in coronary arteries, renal arteries, peripheral arteries including illiac arteries, arteries of the neck and cerebral arteries. The stents 100 of the present invention, however, are not limited to use in the vascular system and may also be advantageously employed in other body structures, including but not limited to arteries, veins, biliary ducts, urethras, fallopian tubes, bronchial tubes, the trachea, the esophagus and the prostate.

## Claims

1. A stent (100) comprising:
a first segment comprising a plurality of closed serpentine circumferential bands (110), adjacent closed serpentine circumferential bands connected to one another, each closed serpentine circumferential band comprising a plurality of struts (112), struts (112) which are circumferentially adjacent one another connected one to the other by a turn (114), each strut having a length, the struts generally increasing in length from a minimum strut length (112a) to a maximum strut length (112b) and then generally decreasing in length from the maximum strut length (112b) to the minimum strut length (112a) as the circumferential band is traversed in its entirety in a clockwise direction, **characterised in that** the struts of maximum length in the closed serpentine bands are longitudinally aligned with one another, and the struts of a closed serpentine band continually increase in length from a minimum strut length to a maximum strut length and then continually decrease in length from the maximum strut length to the minimum strut length as the closed serpentine band is traversed in its entirety in a clockwise direction.

2. The stent of claim 1, wherein the struts of each closed serpentine band continually increase in length from a minimum strut length to a maximum strut length and then continually decrease in length from the maximum strut length to the minimum strut length as the closed serpentine band is traversed in its entirety in a clockwise direction.

3. The stent of claim 1 wherein the struts of maximum length are arranged in a longitudinal strip.

4. The stent of claim 1, further comprising at least one segment of a different geometry from the first segment.

5. The stent of claim 1 wherein each closed serpentine circumferential band has a first end and a second end and the turns at only one of the first and second ends are in general circumferential alignment, the turns at the other end being non-aligned circumferentially.

6. The stent of claim 5 including closed serpentine bands having turns which are in general circumferential alignment at the first ends of the closed serpentine bands and turns which are non-aligned circumferentially at the second ends; and including closed serpentine bands having turns which are in general circumferential alignment at the second ends of the closed serpentine bands and turns which are non-aligned circumferentially at the first ends.

7. The stent of claim 6 including two closed serpentine bands which are adjacent one another, one of the two closed serpentine bands having circumferentially nonaligned turns at the second end, the other of the two closed segments having circumferentially non-aligned turns at the first end, the circumferentially non-aligned turns of the two closed serpentine bands facing one another.

8. The stent of claim 1 including one or more closed serpentine bands whose turns at a first end are circumferentially non-aligned and whose turns at a second end are circumferentially non-aligned.

9. The stent of claim 1 wherein each of the closed serpentine bands has circumferentially non-aligned turns at a first end of the band and circumferentially nonaligned peaks at a second end of the band.

10. The stent of claim 1 wherein the struts of minimum length in the closed serpentine bands are generally longitudinally aligned with one another.

11. The stent of claim 1 wherein at least one connecting element comprises a curved portion.

12. The stent of claim 11 wherein at least one connecting element includes a peak and a valley.

13. The stent of claim 1, wherein a first connecting element has a greater length than a second connecting element, the first connecting element being circumferentially adjacent to the second connecting element.

## Patentansprüche

1. Stent (100), umfassend:
eine erstes Segment, umfassend eine Vielzahl von geschlossenen serpentinenförmigen umlaufenden Bändern (110), wobei aneinander angrenzende geschlossene serpentinenförmige umlaufende Bänder miteinander verbunden sind, jedes geschlossene serpentinenförmige umlaufende Band eine Vielzahl von Streben (112) umfasst, Streben (112) welche in Umfangsrichtung aneinander angrenzend sind miteinander durch eine Kurve (114) verbunden sind, jede Strebe eine Länge hat, wobei die Streben allgemein von einer minimalen (112a) Strebenlänge zu einer maximalen (112b) Strebenlänge an Länge zunehmen und dann von der maximalen (112b) Strebenlänge zu der minimalen (112a) Strebenlänge an Länge abnehmen, wenn das umlaufende Band im Uhrzeigersinn in seiner Gesamtheit durchquert wird, **dadurch gekennzeichnet dass** die Streben von maximaler Länge in den geschlossenen serpentinenförmigen Bändern in Längsrichtung zueinander ausgerichtet sind und die Streben eines geschlossenen serpentinenförmigen Bandes von einer minimalen Strebenlänge zu einer maximalen Strebenlänge kontinuierlich an Länge zunehmen und dann von der maximalen Strebenlänge zu der minimalen Strebenlänge kontinuierlich an Länge abnehmen, wenn das geschlossene serpentinenförmige Band im Uhrzeigersinn in seiner Gesamtheit durchquert wird.

2. Stent gemäß Anspruch 1, wobei die Streben von jedem geschlossenen serpentinenförmigen Band von einer minimalen Strebenlänge zu einer maximalen Strebenlänge kontinuierlich an Länge zunehmen und dann von der maximalen Strebenlänge zu der minimalen Strebenlänge kontinuierlich an Länge abnehmen, wenn das geschlossene serpentinenförmige Band im Uhrzeigersinn in seiner Gesamtheit durchquert wird.

3. Stent gemäß Anspruch 1, wobei die Streben von maximaler Länge in einem längsgerichteten Streifen angeordnet sind.

4. Stent gemäß Anspruch 1, ferner umfassend mindestens ein Segment mit einer Geometrie, die sich von derjenigen des ersten Segmentes unterscheidet.

5. Stent gemäß Anspruch 1, wobei jedes geschlossene serpentinenförmige Band ein erstes Ende und ein zweites Ende hat und die Kurven an nur einem aus dem ersten und dem zweiten Ende in allgemeiner Ausrichtung in Umfangsrichtung sind, wobei die Kurven an dem anderen Ende nicht in Umfangsrichtung ausgerichtet sind.

6. Stent gemäß Anspruch 5, beinhaltend geschlossene serpentinenförmige Bänder mit Kurven, welche an den ersten Enden der geschlossenen serpentinenförmigen Bänder in allgemeiner Ausrichtung in Umfangsrichtung sind, sowie an den zweiten Enden Kurven, welche nicht in Umfangsrichtung ausgerichtet sind; und beinhaltend geschlossene serpentinenförmige Bänder mit Kurven, welche an den zweiten Enden der geschlossenen serpentinenförmigen Bänder in allgemeiner Ausrichtung in Umfangsrichtung sind, sowie an den ersten Enden Kurven, welche nicht in Umfangsrichtung ausgerichtet sind.

7. Stent gemäß Anspruch 6, beinhaltend zwei geschlossene serpentinenförmige Bänder die aneinander angrenzen, wobei eines der beiden geschlossenen serpentinenförmigen Bänder in Umfangsrichtung nicht ausgerichtete Kurven an dem zweiten Ende aufweist, die anderen der beiden geschlossenen Segmente in Umfangsrichtung nicht ausgerichtete Kurven an dem ersten Ende aufweisen, wobei die in Umfangsrichtung nicht ausgerichteten Kurven der beiden geschlossenen serpentinenförmigen Bänder einander zugewandt sind.

8. Stent gemäß Anspruch 1, beinhaltend eines oder mehrere geschlossene serpentinenförmige Bänder, deren Kurven an einem ersten Ende in Umfangsrichtung nicht ausgerichtet sind, und deren Kurven an einem zweiten Ende in Umfangsrichtung nicht ausgerichtet sind.

9. Stent gemäß Anspruch 1, wobei jedes der geschlossenen serpentinenförmigen Bänder in Umfangsrichtung nicht ausgerichtete Kurven an einem ersten Ende des Bandes und in Umfangsrichtung nicht ausgerichtete Gipfel an einem zweiten Ende des Bandes aufweist.

10. Stent gemäß Anspruch 1, wobei die Streben von minimaler Länge in den geschlossenen serpentinenförmigen Bändern allgemein in Längsrichtung zueinander ausgerichtet sind.

11. Stent gemäß Anspruch 1, wobei mindestens ein Verbindungselement einen gekrümmten Abschnitt umfasst.

12. Stent gemäß Anspruch 11, wobei mindestens ein Verbindungselement einen Gipfel und ein Tal beinhaltet.

13. Stent gemäß Anspruch 1, wobei ein erstes Verbindungselement eine größere Länge als ein zweites Verbindungselement aufweist und wobei das erste Verbindungselement in Umfangsrichtung an das zweite Verbindungselement angrenzt.

## Revendications

1. Stent (100), comprenant:
un premier segment, comprenant une pluralité de bandes circonférentielles en serpentin fermées (110), des bandes circonférentielles en serpentin fermées adjacentes étant reliées l'une à l'autre, chaque bande circonférentielle en serpentin fermée comprenant une pluralité d'entretoises (112), des entretoises (112) qui sont circonférentiellement adjacentes l'une à l'autre étant reliées l'une à l'autre par une courbe (114), chaque entretoise ayant une longueur, les entretoises généralement accroissant en longueur d'une longueur d'entretoise minimale (112a) à une longueur d'entretoise maximale (112b), et ensuite diminuant en longueur de la longueur d'entretoise maximale (112b) à la longueur d'entretoise minimale (112a) quand la bande circonférentielle est traversée au sens des aiguilles de montre dans son entièreté, **caractérisé en ce que** les entretoises de longueur maximale dans les bandes circonférentielles en serpentin fermées sont longitudinalement alignées l'une à l'autre et les entretoises d'une bande circonférentielle en serpentin fermée accroissent en longueur de façon continue d'une longueur d'entretoise minimale à une longueur d'entretoise maximale, et ensuite diminuent en longueur d'entretoise de façon continue de la longueur d'entretoise maximale à la longueur d'entretoise minimale quand la bande circonférentielle en serpentin fermée est traversée au sens des aiguilles de montre dans son entièreté.

2. Stent selon la revendication 1, dans lequel les entretoises de chaque bande circonférentielle en serpentin fermée accroissent en longueur de façon continue d'une longueur d'entretoise minimale à une longueur d'entretoise maximale, et ensuite diminuent en longueur d'entretoise de façon continue de la longueur d'entretoise maximale à la longueur d'entretoise minimale quand la bande circonférentielle en serpentin fermée est traversée au sens des aiguilles de montre dans son entièreté.

3. Stent selon la revendication 1, dans lequel les entretoises de longueur maximale sont disposées dans un ruban longitudinal.

4. Stent selon la revendication 1, comprenant en outre au moins un segment d'une géométrie différente du premier segment.

5. Stent selon la revendication 1, dans lequel chaque bande circonférentielle en serpentin fermée a une première extrémité et une deuxième extrémité et les courbes dans seulement une des extrémités première et deuxième sont en alignement circonférentiel général, les courbes dans l'autre extrémité n'étant pas alignées circonférentiellement.

6. Stent selon la revendication 5, incluant des bandes circonférentielles en serpentin fermées avec des courbes qui sont en alignement circonférentiel général dans les premières extrémités des bandes circonférentielles en serpentin fermées, et des courbes qui ne sont pas alignées circonférentiellement dans les deuxièmes extrémités; et incluant des bandes circonférentielles en serpentin fermées avec des courbes qui sont en alignement circonférentiel général dans les deuxièmes extrémités des bandes circonférentielles en serpentin fermées, et dans les premières extrémités des courbes qui ne sont pas alignées circonférentiellement.

7. Stent selon la revendication 6, incluant deux bandes circonférentielles en serpentin fermées adjacentes l'une à l'autre, une des deux bandes circonférentielles en serpentin fermées ayant des courbes non alignées circonférentiellement dans la deuxième extrémité, les autres des deux segments fermés ayant des courbes non alignées circonférentiellement dans la première extrémité, les courbes non alignées circonférentiellement des deux bandes circonférentielles en serpentin fermées étant vis-à-vis l'une à l'autre.

8. Stent selon la revendication 1, incluant une ou plusieurs bandes circonférentielles en serpentin fermées, dont les courbes ne sont pas alignées circonférentiellement dans une première extrémité et dont les courbes ne sont pas alignées circonférentiellement dans une deuxième extrémité.

9. Stent selon la revendication 1, dans lequel chacune des bandes circonférentielles en serpentin fermées a des courbes non alignées circonférentiellement dans une première extrémité de la bande et des sommets non alignées circonférentiellement dans une deuxième extrémité de la bande.

10. Stent selon la revendication 1, dans lequel les entretoises de longueur minimale dans les bandes circonférentielles en serpentin fermées sont généralement longitudinalement alignées l'une à l'autre.

11. Stent selon la revendication 1, dans lequel au moins un élément connecteur comprend une partie courbée.

12. Stent selon la revendication 11, dans lequel au moins un élément connecteur comprend un sommet et une vallée.

13. Stent selon la revendication 1, dans lequel un premier élément connecteur a une plus grande longueur qu'un deuxième élément connecteur, le premier élément connecteur étant circonférentiellement adjacent au deuxième élément connecteur.
